# EUROPEAN PATENT SPECIFICATION

(11) **EP 0 437 378 B1**
(45) Date of publication and mention of the grant of the patent: **19.05.1999**
(21) Application number: 91300225.9
(22) Date of filing: 11.01.1991
(51) Int. Cl.: C12N 15/54, C12N 1/19, C12N 1/21

(54) **DNA sequence for an antibiotic biosynthesis enzyme**
Für ein Enzym der Antibiotika-Biosynthese kodierende DNA-Sequenz
Séquence d'ADN codant pour un enzyme de la biosynthèse d'un antibiotique

(30) Priority: 12.01.1990 GB 9000699; 20.04.1990 GB 9008876
(43) Date of publication of application: 17.07.1991
(73) Proprietor: GLAXO GROUP LIMITED, Greenford, Middlesex UB6 0NN (GB)
(72) Inventor: Maishman, Nicholas John, Ulverston, Cumbria, LA12 9 DR (GB); Ramsden, Martin, Ulverston, Cumbria, LA12 9 DR (GB)
(74) Representative: James, Stephen Richard, Dr.

(56) References cited:
- EP-A- 200 425
- EP-A- 281 391
- EP-A- 0 450 758
- JOURNAL OF BIOTECHNOLOGY Vol. 3, 1985, Amsterdam NL, pages 109-117; A. SCHEIDEGGER et al.: "Investigation of acetyl-CoA: Deacetylcephalosporin C-acetyltransferase of Cephalosporium acremonium"

## Description

The present invention relates to novel DNA sequences and, in particular, to DNA or a restriction fragment derived therefrom comprising a DNA sequence coding for DAC acetyltransferase activity. This enzyme activity is involved in the biosynthesis of β-lactam antibiotics.

The enzyme deacetylcephalosporin C (DAC) acetyltransferase catalyses the final step in the biosynthetic pathway of cephalosporin C, shown below:

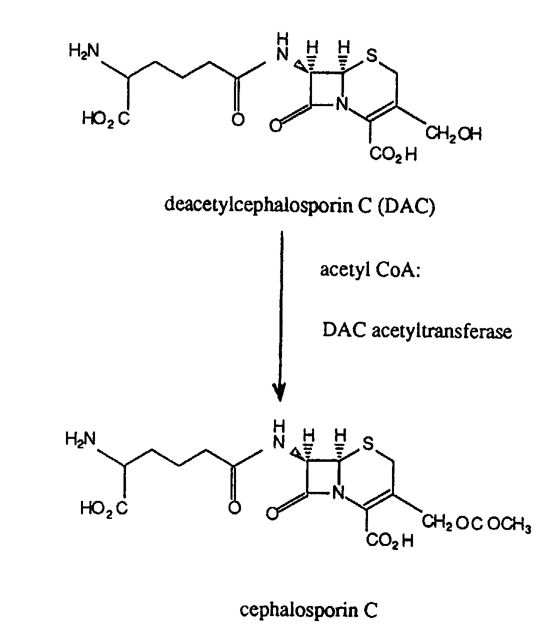

Although cephalosporin C has been shown to have considerable activity againist gram-negative microorganisms as an antibiotic itself, the major commercial use of cephalosporin C is as a building-block for other cephalosporin-like antibiotics. In particular, the D-α-aminoadipoyl side chain may be removed by chemical cleavage of the amide link to give the highly useful intermediate 7-aminocephalosporanic acid (7-ACA) which is a percursor to a wide range of cephalosporin antibiotics such as ceftazidime and cefuroxime.

Traditional methods of strain improvements have been time consuming and laborious involving standard methods of mutagenesis followed by lengthy screening for improved antibiotic titre of the strains thus produced. Such methods are inefficient and have a low degree of success. A more directed method of maximising the efficiency of the biosynthesis of antibiotic precursors involves the introduction of multiple copies of a gene coding for a biosynthesis pathway enzyme. Another method is to introduce one or more copies of a biosynthesis enzyme gene which has been altered to improve its expression making it more effective. Such directed modification may be achieved, for example, by combining the gene with a more efficient promoter. The isolation of the DNA sequences of the invention, however, will enable the efficient introduction of multiple and/or modified copies of a DAC acetyltransferase encoding DNA sequence and hence the potential accumulation of higher intracellular levels of cephalosporin C thereby improving the productivity and cost-effectiveness of antibiotic fermentation from transformed hosts.

Further advantages that may be derived for the isolation of DNA sequences of the invention include potential improved enzyme stability of the DAC acetyltransferase in a transformed host and improved enzyme activity. The isolated DNA sequences of the invention might also enable the enzyme to be produced in different hosts for the production of related cephalosporins in other antibiotic-producing organisms, possibly producing in this way heretofor unknown metabolites.

J. Biotechnol., Vol 3, p 109-117, 1985, discloses a partially purified preparation of the DAC acetyltransferase enzyme from Acremonium chrysogenum.

EP-A-0 450 758 discloses DNA coding for DAC acetyltransferase. It has a priority date after that of the present invention.

European Patent Publication No. 0281391 discloses recombinant DNA expression vectors and DNA compounds that encode deacetoxycephalosporin C synthetase (DAOCS) and deacetylcephalosporin C synthetase (DACS), the cephalosporin biosynthetic pathway enzymes which together comprise the so-called "expandase/hydroxylase" activity. The DNA compound that encodes the DAOCS/DACS activity was isolated from Acremonium chrysogenum (formerly known as Cephalosporium acremonium) genomic DNA as a ∼7 kb DNA compound. A 2.2 kb PstI-BamHI DAOCS/DACS-encoding restriction fragment was used to prepare recombinant DNA expression vectors for the expression of expandase/hydroxylase activity.

We have now found that the gene encoding DAC acetyltransferase is located on another portion of the -7 kb DNA compound. Using a similar DNA sequence to that disclosed in European Patent Publication No. 0281391, we have now isolated DNA sequences encoding DAC acetyltransferase activity.

Accordingly, there is provided in a first aspect of the present invention a recombinant DNA expression vector comprising a recombinant DNA sequence encoding DAC acetyltransferase activity obtainable from Acremonium chrysogenum, wherein said recombinant DNA sequence does not contain a gene coding on expression for the native DAOCS/DACS polypeptide, and encodes a protein comprising as its amino terminal the structure shown in SEQ ID NO:1.

In an alternative aspect of the present invention, there is provided a recombinant DNA sequence or a restriction fragment thereof containing a gene coding on expression for DAC acetyltransferase activity obtainable from Acremonium chrysogenum, but not containing a gene coding on expression for the native DAOCS/DACS polypeptide, and encoding a protein comprising as its amino terminal the structure shown in SEQ ID NO:1.

In a further alternative aspect of the present invention, there is provided a recombinant DNA expression vector comprising a DNA sequence encoding DAC acetyltransferase activity obtainable from Acremonium chrysogenum, wherein said DNA sequence does not encode DAOCS/DACS activity, and encodes a protein comprising as its amino terminal the structure shown in SEQ ID NO:1.

In an alternative aspect of the present invention, there is provided a recombinant DNA sequence or a restriction fragment thereof containing the gene coding on expression for DAC acetyltransferase activity obtainable from Acremonium chrysogenum, but not containing the gene coding on expression for DAOCS/DACS activity, and encoding a protein comprising as its amino terminal the structure shown in SEQ ID NO:1.

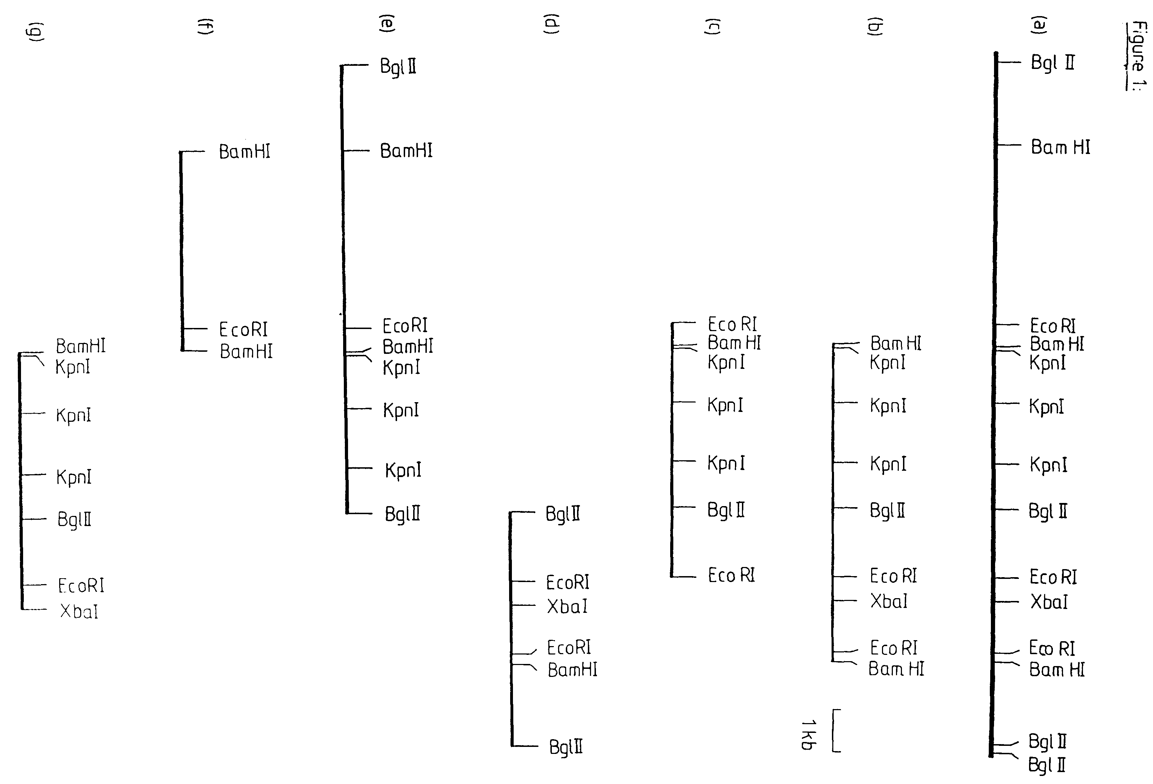

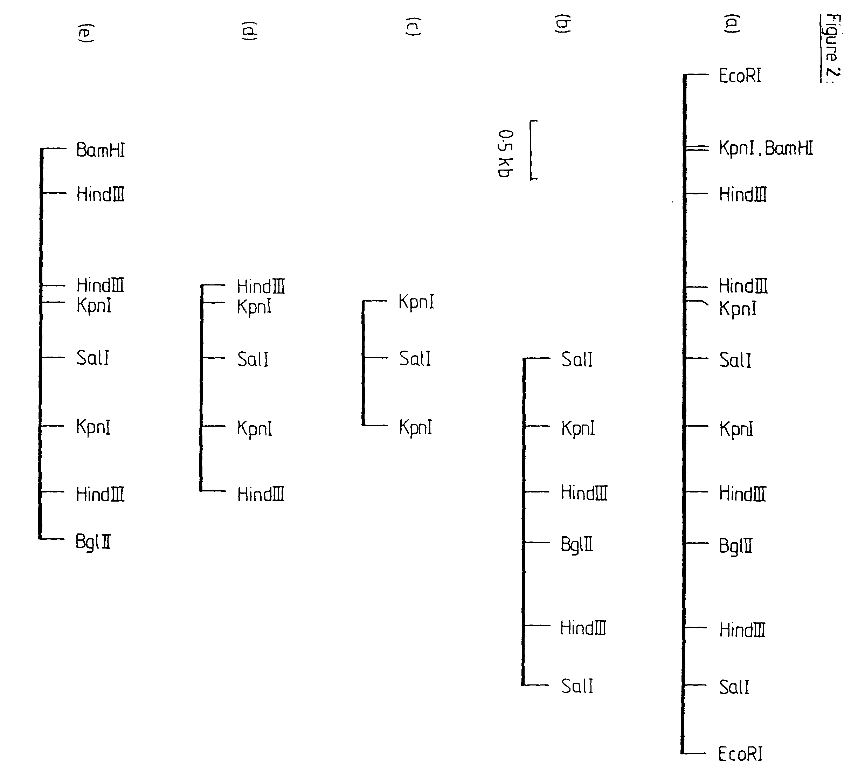

Recombinant DNA sequences containing the gene coding on expression for DAC acetyltransferase activity but not a gene coding on expression for the native DAOCS/DACS polypeptide are, for example, shown in Figure 1(c), 1(e), 2(a) and 2(b).

The DNA sequences of Figure 1(c) and 2(a) are the same and contain ∼5.8kb. The DNA sequence of Figure 1(e) contains ∼9kb and figure 2(b) contains ∼2.8kb.

In a further aspect of the present invention, there is provided a recombinant DNA expression vector comprising a recombinant DNA sequence of the invention selected from the ∼5.8kb EcoRI restriction fragment of plasmids pUX3 or pUX4, the ∼2.8kb Sal I restriction fragment of plasmid pUX9, or the ∼9kb BglII restriction fragment of plasmid pUX11.

It will be appreciated that the recombinant DNA expression vector may be selected from one of the plasmids pUX3, pUX4, pUX9 and pUX11.

It is not intended that the DNA sequences of the present invention incorporate the sequences as they occur in nature. The term "recombinant DNA" as used herein includes DNA sequences of whatever origin otherwise than as they occur in nature.

By the term "DAC acetyltransferase activity" is meant any polypeptide which is capable of catalysing the acetylation of deacetylcephalosporin C to cephalosporin C.

The term "native DAOCS/DACS polypeptide" as used herein means the unaltered polypeptide sequence as isolated from Acremonium chrysogenum. It is not intended to include any modified or improved polypeptide sequence which may also exhibit DAOCS/DACS activity.

A "gene coding on expression for the native DAOCS/DACS polypeptide" may be either the DAOCS/DACS gene as isolated from Acremonium chrysogenum or a homologue thereof differing from the native DAOCS/DACS gene only in the degenerate nature of the genetic code and thus coding on expression for the unaltered DAOCS/DACS polypeptide sequence.

It will be appreciated that a polypeptide with "DAC acetyltransferase activity" may accept substrates other than deacetylcephalosporin C, for example, other antibiotics or antibiotic precursors where the 3-position substituent on the cephem ring is desirably converted from -CH₂OH to -CH₂OCOCH₃.

In one preferred aspect the recombinant DNA sequences of the present invention encode a polypeptide which comprises an amino acid sequence substantially corresponding to the sequence of native DAC acetyltransferase as found in A. chrysogenum.

The sequence of a polypeptide with DAC acetyltransferase activity according to the invention will thus be identical to the sequence of a native DAC acetyltransferase enzyme or contain one or more deletions, substitutions, insertions, inversions or additions of allelic origin or otherwise, the resulting polypeptide will have at least 80% and preferably 90% homology with the sequence of a native DAC acetyltransferase enzyme and retain essentially the same DAC acetyltransferase activity as herein defined.

It will be appreciated that the DAC acetyltransferase-encoding DNA sequences of the present invention may comprise an operatively linked transcription and translation activating sequence that controls the expression of the DAC acetyltransferase-encoding DNA sequence. Such activating sequences for use in recombinant DNA expression are well known in the art although, where the intended host is the same species as that from which the recombinant DNA sequences of the invention were isolated, the activating sequence is preferably isolated together with the DAC acetyltransferase-encoding DNA sequences to avoid the use of foreign activating sequences. Alternatively, the recombinant DNA sequences of the invention may be combined with alternative activating sequences by conventional methods to express that sequence or the portions thereof that encode DAC acetyltransferase activity.

It will also be appreciated that it is particularly desirable to combine the DAC acetyltransferase-encoding DNA sequence with an ATG start codon by conventional methods.

It will be further appreciated that the DAC acetyltransferase- encoding DNA sequences of the present invention may comprise regulatory signals at the 3' end of the coding sequence such as the stop codons TAG, TAA and TGA, and mRNA polyadenylation and processing signals.

The term "DAC acetyltransferase-encoding DNA sequences" is intended to incorporate those sequences which contain one or more modifications such as mutations, including single or multiple base substitutions, deletions, insertions or inversions and which code on expression for a polypeptide possessing DAC acetyltransferase activity as herein defined. As used herein, the term DAC acetyltransferase-encoding DNA sequences does not include DNA sequences which additionally contain a gene which also codes on expression for the native DAOCS/DACS polypeptide. Where modified DNA sequences are used, particularly preferred are those recombinant DNA sequences which are degenerate as a result of the genetic code which code on expression for a polypeptide with an amino acid sequence identical to that of a native DAC acetyltransferase enzyme.

It will further be appreciated that the DAC-acetyltransferase-encoding sequences of the present invention may be combined with other recombinant DNA sequences coding on expression for other β-lactam antibiotic biosynthesis enzymes. Thus, for example, the DAC-acetyltransferase-encoding sequences of the present invention may be combined with a recombinant DNA sequence coding on expression for a mutant DAOCS/DACS polypeptide which varies from the native DAOCS/DACS polypeptide at any number of amino acid residue positions. Such mutant polypeptides would be encoded by mutant DAOCS/DACS -encoding DNA sequences, including sequences in which amino acid codons have been deleted from, inserted into, or replaced in the native DAOCS/DACS coding sequence.

In a further aspect of the invention there is provided a recombinant DNA expression vector comprising a recombinant DNA sequence and additionally comprising a recombinant DNA sequence coding on expression for a mutant DAOCS/DACS polypeptide. Such recombinant DNA sequences may be operatively linked to promoters and translation activating sequences, for example, as described herein.

The recombinant DNA sequences of the present invention are of use in the modification of an antibiotic producing microorganism to introduce or improve its DAC acetyltransferase activity. As used herein, the term "antibiotic-producing microorganism" extends to any organism including, but not limited to, species that either produce a β-lactam antibiotic, contain genes that, if expressed, would produce a β-lactam antibiotic, or are deficient in one or more genes required to confer upon the microorganism the ability to produce β-lactam antibiotics. As used herein, the term "β-lactam antibiotic" refers in particular to a cephalosporin antibiotic.

The recombinant DNA sequences of the present invention may be introduced directly into the genome of the antibiotic-producing microorganism in which case they may be used as such. Alternatively, the recombinant DNA sequences of the invention may be introduced by standard techniques known in the art by the use of recombinant DNA expression vectors. When introduced in this way it will be appreciated that the vector will, in general, additionally comprise promoter and/or translation activating sequences, operatively linked to the DAC acetyltransferase-encoding DNA sequences and preferably additionally using a selectable marker system for identifying the transformed host, for example, as described herein below.

There is thus provided in a further aspect of the present invention a recombinant DNA expression vector comprising a recombinant DNA sequence of the invention and additionally comprising a promoter and translation activating sequence operatively linked to the DAC acetyltransferase-encoding DNA sequence.

In an alternative aspect the recombinant DNA sequences of the present invention may be introduced into a non-antibiotic-producing microorganism, for example, Escherichia coli, either directly or as part of a recombinant DNA expression vector to provide the expression and isolation of an enzyme with DAC acetyltransferase activity which may be useful in catalysing in vitro antibiotic preparation or the in vitro preparation of precursors for use in antibiotic production. Even if expression of a polypeptide with DAC acetyltransferase activity is not intended in E.coli, a suitable recombinant DNA expression vector should preferably be capable of replication in a prokaryotic host such as E.coli to facilitate further genetic manipulation.

According to a further aspect of the present invention, there is thus provided a eukaryotic or prokaryotic host transformed with a recombinant DNA expression vector comprising a DAC acetyltransferase-encoding DNA sequence of the invention.

Preferred hosts include species of filamentous fungi such as Acremonium spp or Penicillium spp, species of streptomyces, or bacteria such as E.coli or Bacillus spp. Particularly preferred hosts are Acremonium chrysogenum or Penicillium chrysogenum.

In a further aspect of the present invention, there is provided a method of expressing DAC acetyltransferase activity in a eukaryotic or prokaryotic host comprising:
a) transforming the host cell with a recombinant DNA expression vector that comprises (i) an expression control sequence that functions in the host cell, and (ii) a DAC acetyltransferase- encoding DNA sequence which is operatively linked to the expression control sequence;
b) culturing the host cell transformed in step (a) under conditions that allow for expression of DAC acetyltransferase activity.

It will be appreciated that the DNA sequence described herein may contain lengths of DNA which do not code on expression for DAC acetyltransferase. Although these non-coding sequences in no way detract from the usefulness of the whole recombinant DNA sequence, it may be convenient or desirable to utilise a smaller restriction fragment derived from the recombinant DNA sequences. The restriction fragments may be obtained, for example, by cleavage with suitable restriction enzymes by methods well known in the art.

Suitable recombinant DNA expression vectors may comprise chromosomal, non-chromosomal and synthetic DNA such as derivatives of known bacterial plasmids, for example, 'natural' plasmids such as ColE1 or 'artificial' plasmids such as pBR322, pAT153, pUC18, pUC19, pACYC 184 or pMB9, or yeast plasmids, for example, 2µ. Other suitable vectors may be phage DNA's, for example, derivatives of M13, or bacteriophage lambda (λ), or yeast vectors such as derivatives of YIp, YEp or YRp.

It will be appreciated that apart from containing the recombinant DNA sequence or a restriction fragment derived therefrom as herein defined, the recombinant DNA expression vector may also comprise a promoter and translational activating sequence which not only functions in the chosen host cell, but also is operatively linked - meaning that it is positioned in the correct orientation and position to control expression of the DAC acetyltransferase-encoding DNA sequence. Such an activating sequence may, of course, be found in the full-length DNA molecule described herein.

Examples of useful promoter sequences which might be used in the recombinant DNA expression vectors of the invention include, for example, the pcbC promoter of isopencillin N synthetase (IPNS) from Acremonium spp or Penicillium spp, the cefEF promoter of expandase/hydroxylase (DAOCS/DACS) from Acremonium spp, the penDE promoter of Acyl CoA:6APA acyl transferase from Penicillium spp, and numerous promoter sequences from Aspergillus spp including pgk, gpdA, pki, amdS, argB, trpC, alcA, aldD and niaD promoter sequences.

Further examples of useful promoter sequences which might be used in the recombinant DNA expression vectors of the invention include the glycolytic promoters of yeast (for example, the promoter of 3-phosphoglycerate kinase, PGK), the promoters of yeast acid phosphatase (for example, Pho 5) or the alcohol dehydrogenase-2 (alcA) or glucoamylase promoter.

Preferably the control sequence is isolated with the DAC acetyltransferase-encoding DNA thus avoiding the need to construct the vector using foreign control sequences.

In addition, such recombinant DNA expression vectors may possess various sites for insertion of the DAC acetyltransferase-encoding DNA sequences of this invention. These sites are characterised by the specific restriction endonuclease which cleaves them. Such cleavage sites are well recognised by those skilled in the art. The expression vector, and in particular the site chosen therein for insertion of a selected DNA fragment and its operative linking to an expression control sequence, is determined by a variety of factors including the number of sites susceptible to a given restriction enzyme, the size of the protein to be expressed, contamination or binding of the protein to be expressed by host cell proteins which may be difficult to remove during purification, the location of start/stop codons, and other factors recognised by those skilled in the art. Thus the choice of a vector and insertion site for a recombinant DNA sequence is determined by a balance of these factors, not all selections being equally effective for a give case. Likewise, not all host/vector combinations will function with equal efficiency in expressing the recombinant DNA sequences of this invention. The selection is made, depending upon a variety of factors including compatability of the host and vector, ease of recovery of the desired protein and/or expression characteristics of the recombinant DNA sequences and the expression control sequences operatively linked to them, or any necessary post-expression modifications of the desired protein.

The recombinant DNA expression vector may also comprise a selectable marker which will facilitate screening of the chosen host for transformants. Suitable markers for use in fungal transformations are well known in the art and include the acetamidase (amdS) gene which confers upon transformants the ability to utilise acetamide as the sole source of nitrogen, antibiotic resistance, for example, the phosphotransferase genes which confer upon transformants resistance to aminoglycosidic antibiotics such as G418, hygromycin B and phleomycin, or resistance to benomyl by transformation with an altered tubulin gene. Another useful marker is the nitrate reductase (niaD) gene which confers upon nitrate reductase deficient mutants the ability to utilise nitrate as the sole nitrogen source. Other marker systems for filamentous fungi involve the reversions of arginine auxotrophy using the argB gene, tryptophan C auxotrophy using the trpC gene and uracil auxotrophy using the pyr-4 or pyrG genes.

It will be appreciated that the DAC acetyltransferase gene of the present invention and the chosen marker gene may conveniently be part of the same plasmid or alternatively they may be on different plasmids in which case the host must be co-transformed using any suitable technique well known in the art.

The recombinant DNA expression vector may also optionally be characterised by an autonomous replication sequence (ars) derived from a fragment of either chromosomal or mitochondrial DNA, preferably from the same species as that being transformed.

To prepare the recombinant DNA sequences of the invention, a random array of DNA fragments may be generated by the partial digestion of genomic DNA from an antibiotic producing strain of A.chrysogenum, using a restriction enzyme such as Sau3A1. [see for instance Garber and Yoder, Anal. Biochem., 135, p416 (1983)]. The DNA fragments thus obtained may then be size fractionated on a sucrose density gradient and fragments in the range of 3 to 30kb, preferably between 5 and 25kb, may be isolated. The digestion fragments may be ligated into a plasmid, for example, pAT153, or a phage vector such as λEMBL3 or λEMBL4. These may then be screened by conventional methods using either all, or a fragment, of a DAC acetyltransferase-encoding DNA sequences derived for example, from the amino acid sequence of a DAC acetyltransferase enzyme from the same or a related species (for a full discussion of recombinant DNA techniques see Old and Primrose, Principles of Gene Manipulation, 3rd Edition, Blackwell Scientific Publications, 1985). One such suitable fragment of the DAC acetyltransferase gene is a KpnI-KpnI fragment which was deposited in a bacteriophage as the clone M13K18/4 at the National Collection of Industrial and Marine Bacteria, Aberdeen, Scotland on 15th December 1989, with the Accession Number NCIMB 40236 under the terms of the Budapest treaty.

Alternatively the cloned DNA may be ligated into recombinant DNA expression vectors as described herein and used to transform, for example a filamentous fungal strain which would produce cephalosporin C but for a deficiency in the activity of DAC acetyltransferase. Such strains are therefore unable to produce cephalosporin C since the biosynthetic pathway is blocked and are thus readily distinguished from strains transformed by the DNA sequences of the invention which will have a DAC acetyltransferase-encoding DNA sequence and will therefore produce cephalosporin C.

Suitable strains may arise with low frequency by spontaneous mutation, or alternatively, mutation may be induced by standard mutagenesis techniques well known in the art. One particularly useful strain is an A. strictum strain (ATCC 20371) which is a DAC overproducer and makes very little cephalosporin C.

Screening for transformants involves the identification of those transfectants which produce cephalosporin C. Such a characteristic would be indicative of a reversion of the deficiency in DAC acetyltransferase due to the replacement of the missing biosynthetic pathway gene. Therefore a cephalosporin C sensitive microorganism, for example, Alcaligenes faecalis (ATCC 8750), is conveniently grown on agar plates and a complementation assay may be conducted by inserting agar plugs of the transformants into the plates by conventional methods known in the art and identifying 'positive' transformants by their ability to inhibit growth of the sensitive microorganism.

The recombinant DNA expression vectors may be recovered from each 'positive' transformants by conventional techniques and the DAC acetyltransferase encoding DNA sequence characterised by cleavage with a variety of restriction endonuclease enzymes. Such a technique will readily identify recombinant DNA sequences of the present invention.

One such recombinant DNA sequence of the invention is the BamHI-BglII fragment of Figure 2e which was deposited in a plasmid in E.coli at the National Collection of Industrial and Marine Bacteria, Aberdeen, Scotland on 27th February 1990, with the Accession Number NCIMB 40264 under the terms of the Budapest Treaty.

The first 459 nucleotides of the cefG gene coding for the DAC acetyltransferase enzyme are given in Sequence ID No 1.

It will be appreciated that characteristic restriction sites may be introduced or deleted in the DNA, or fragments derived therefrom, of the present invention. Such modifications may conveniently be effected by the method of site directed mutagenesis as described, for example, by Zoller and Smith, Nucl.Acids Res., 10, p6487 (1982), thus enabling further genetic manipulation of the DNA.

Techniques such as site directed mutagenesis may be used to make specific insertions and/or deletions resulting in a mutated DNA which may be used to obtain an improved antibiotic titre of known β-lactam antibiotics from a suitable host. In addition, mutant DNA may also be used to obtain modified enzymes (muteins) which may recognise unnatural substrates and thus produce unusual and/or unnatural cephalosporins.

In the following non-limited examples which illustrate the present invention, the following abbreviations are used:-
DAC - deacetylcephalosporin C; DAT - DAC acetyltransferase; EDTA - ethylenediaminetetraacetic acid; TE - Tris/EDTA; Tris-HCl - tris(hydroxymethyl)aminomethane hydrochloride; SDS - sodium dodecyl sulphate; DNase - deoxyribonuclease; RNase - ribonuclease; ATP - adenosine triphosphate; DTT - DL-dithiothreitol; Ap - ampicillin; kb - kilobase pairs of DNA; EGTA - ethylene glycolbis(β-aminoethyl ether)-N,N,N',N'-tetraacetic acid; PEG-polyethylene glycol; DMSO - dimethyl sulphoxide; X-gal - 5-bromo-4-chloro-3-indolyl β-D-galactopyranoside; IPTG - isopropyl β-D-thioglucoside.

### Example: Isolation of a DNA Sequence Encoding DAC Acetyltransferase Activity

### Preparation 1: Isolation of A.chrysogenum DNA

(a) A.chrysogenum Strains
   The preferred A.chrysogenum strain is obtainable from American Type Culture Collection under the accession number ATCC 14553. Other strains, for example CMICC 237 183, or commercial strains derived by mutation, selection and screening programmes are also suitable sources of high molecular weight DNA.
(b) Storage and Inoculum Preparation
   An ampoule of spores or mycelial fragments (approximately 2 x 10⁸ colony forming units in 1 ml of preservation medium; 10% methanol) was taken from storage over liquid nitrogen and rapidly thawed under warm water. About 50 µl of this suspension was inoculated onto each agar slope culture as required. The agar slopes consisted of about 10 ml Sabourauds agar (Difco Laboratories, Central Avenue, East Molesey, Surrey KT8 OSE). Cultures were incubated for between 5 - 14 days (dependent on strain growth rate) at 25.5 °C in a humidified atmosphere. About 5 ml of preservation medium was added to each slope, then spores and/or mycelial fragments were suspended by gentle scraping with a sterile inoculating loop. The material was aliquotted as appropriate for long term storage over liquid nitrogen.
(c) Growth of A.chrysogenum in Liquid Culture
   Viable cells from a preserved ampoule or a fresh agar slope culture were inoculated into 100 ml growth medium in a 500 ml flask. The medium, a modification of Demain medium (Queener et al, Microbiology 1985 (Ed., L.Leive) pp 468-472. American Society for Microbiology, Washington DC 1985), comprised: 3.9% sucrose; 0.75% L-asparagine; 1.5% KH₂PO₄; 2.1% K₂HPO₄; 0.075% Na₂SO₄; 0.018% MgSO₄; 0.006% CaCl₂; 0.05% corn steep liquor; 0.022% ammonium acetate; 0.02% CaCO₃; 0.0015% ammonium sulphate; 0.0003% MnSO₄; 0.0003% ZnSO₄; 0.00008% CuSO₄.
   The culture was incubated for 3 - 4 days at 25.5 °C and 220 rpm.
(d) DNA Extraction
   Mycelium was harvested by vacuum filtration on Whatman number 1 filter paper. Thin mycelial pads were quick-frozen in liquid nitrogen, broken into small fragments and lyophilised overnight. This material was used to prepare high molecular weight DNA by a modification of the method of Raeder and Broda (Lett. Appl. Microbiol. 1 (1985): 17 - 20). Lyophilised mycelium (about 2.5 g) was ground to a fine powder with 0.1 volumes of sand, then rehydrated in 5 ml of an extraction buffer consisting of 25 mM EDTA, 0.5% SDS and 250 mM NaCl in 200 mM Tris-HCl, pH 8.5. A 3.5 ml aliquot of phenol (equilibrated with extraction buffer) was added and mixed, followed by 1.5 ml chloroform/isoamyl alcohol (24:1), then the solution was vortexed. The lysate was centrifuged at 12000 g for one hour at room temperature and the aqueous phase recovered to a fresh tube. DNase-free RNase (Gibco-BRL, Renfrew Road, Paisley PA3 4EF) was added to 100 µg/ml and the mixture incubated at 37 °C for 30 minutes.
   Nucleic acids were then precipitated from the aqueous phase with 0.6 vol propan-2-ol. The loose pellet obtained on standing was washed in 1 ml 70% ethanol and raised in 1 ml TE buffer (10 mM Tris-HCl pH 8.0, 1 mM EDTA). Low molecular weight DNA, residual RNA and SDS were precipitated by the addition of 7.5 M ammonium acetate to 2.5 M and incubation at 4 °C for 15 minutes. After a brief centrifugation, as above, DNA was precipitated from the aqueous phase with 2 vol ethanol. The pellet was washed in 70% ethanol, dried and raised in 1 ml TE buffer in about 1 mg yield.

### Preparation 2: A.chrysogenum Genomic Library Construction

(a) Preparation of DNA Fragments
   Aliquots of A.chrysogenum DNA prepared as above (1 µg each) were digested with various levels of the restriction enzyme Sau3AI (0.001-0.1 unit) for various times (10 - 60 minutes) at 37 °C in Sau3A buffer (10 mM Tris-HCl, pH 7.5, 7 mM MgCl₂, 50 mM NaCl). DNA digestion was observed using agarose gel electrophoresis under standard conditions. (See for example, Maniatis T., Fritsch E.F. and Sambrook J.: Molecular Cloning, A Laboratory Manual. Cold Spring Harbour Lab New York, 1982). Fragment sizes were assessed relative to standards (bacteriophage lambda DNA, HindIII and EcoRI fragments). Conditions were chosen which were suitable for digestion of DNA to 15 - 25 kb fragments (0.01 units/µg DNA for 30 - 60 minutes.
   A 200 µg aliquot of the A.chrysogenum DNA was adjusted to Sau3A buffer conditions in a 500 µl volume and incubated at 37 °C with 2 units Sau3AI. At 20, 25, 30, 40, 50 and 60 minutes incubation, a 100 µl aliquot was withdrawn and digestion terminated by addition of EDTA to 10 mM. The aliquots were pooled and the digestion products fractionated by centrifugation through a 38 ml sucrose density gradient (10 - 40% sucrose in 1 M NaCl, 5 mM EDTA, 20 mM Tris-HCl, pH 8.0). Centrifugation was at 22000 rpm, 21 °C for 20 hours in an SW28 rotor (Beckman Instruments (UK) Limited, Progress Road, High Wycombe, Bucks HP12 4JL). The gradient was collected in 0.5 ml fractions, and DNA of size range 15 - 20 kb (as determined by agarose gel electrophoresis) was pooled, adjusted to 0.3 M in sodium acetate and precipitated by addition of 2 volumes ice-cold ethanol and incubation on ice for 30 minutes. The DNA pellet recovered by centrifugation was raised in 25 µl TE buffer.
(b) Preparation of Bacteriophage Library
   The bacteriophage lambda cloning vector λ EMBL3 (Frischauf et al, J. Mol. Biol. 170: (1983) 827 - 842) was obtained as BamHI digested arms from Stratagene Inc (11099 North Torrey Pines Road, La Jolla, CA 92037 USA). The Sau3AI fragments of A.chrysogenum DNA (0.2 µg) and λ EMBL3 arms (1 µg) were mixed in 20 µl ligation buffer (20 mM Tris-HCI, pH 7.6, 10 mM MgCl₂, 10 mM DTT, 0.6 mM ATP) and incubated at 14 °C for 18 hours with 5 units T4 DNA ligase.
   The ligation products were packaged in vitro into phage particles using the commercially available Gigapack kit (Stratagene Inc). The packaged phage were propagated in the widely available E.coli strain Q358, creating a recombinant phage library of 2 x 10⁴ plaque forming units (pfu).

### Preparation 3: Isolation of Clones from the Bacteriophage Library

The recombinant phage library was mixed with 1 x 10⁸ E.coli Q358 cells in 0.5 ml 10 mM MgSO₄. After 15 minutes incubation at 37 °C the mixture was added to 25 ml molten (42 °C) soft agar (LB + 10 mM MgSO₄ + 0.6% agar) and used to overlayer a 20 x 20 cm petri plate containing LB medium + 10 mM MgSO₄ (LB (Luria-Bertani) medium contains 1% Bacto-tryptone, 0.5% yeast extract, 0.5% NaCl at pH 7.0). Standard techniques for handling E.coli are described by Maniatis et al, (loc cit). Following overnight incubation, duplicate impressions of the plate were taken using Hybond-N membranes (Amersham International plc, Amersham, Bucks HP7 9LL) by standard techniques.

The membranes were incubated in 100 ml of pre-hybridisation buffer (6 x SSC, 5 x Denhardt solution, 0.5% SDS and 500 µg denatured salmon sperm DNA) at 65 °C for 6 hours. (1 x SSC is 0.15 M NaCl, 0.015 M sodium citrate; Denhardt solution is 0.02% of each of polyvinyl-pyrrolidone, bovine serum albumin, ficoll (Sigma Chemical Co, Poole, Dorset BH17 7NH).

A pool of 30-mer ogligonucleotides of sequences: 
               T        T
5' GGCAAGTACTCCGACTACTCCACCTGCTAC 3'
               G        G  G
 was synthesised by standard automated means. About 40 pmole of oligonucleotide was incubated in a 100 µl reaction volume containing 40 mM Tris-HCl pH 7.5, 10 mM MgCl₂, 5 mM DTT, 100 pmole (300 µCi) (γ³²P)-ATP and 40 units T4 polynucleotide kinase, at 37 °C for 30 minutes. The reaction was terminated by addition of EDTA to 15 mM followed by addition of 20 µg glycogen (molecular biology grade, Boehringer Corporation London, Bell Lane, Lewes, East Sussex BN7 1LG), sodium acetate to 0.3 M and 2 volumes of cold ethanol. Labelled oligonucleotide was recovered by centrifugation (13000 x g, 10 minutes) washed with 70% v/v cold ethanol, dried and resuspended in 100 ul TE buffer.

The labelled oligonucleotide was added to 80 ml fresh prehybridisation buffer and allowed by hybridise with the membranes at 56 °C overnight. The membranes were washed free of excess label by incubation in 2 changes of about 200 ml 3 x SSC, 0.1% SDS buffer each for 30 minutes at room temperature, followed by 2 changes of 200 ml 3 x SSC, 0.1% SDS buffer each for 20 minutes at 50 °C. Membranes were exposed to X-ray photographic film (Hyperfilm-MP, Amersham International plc) in the presence of intensifying screens at -20 °C for 4 days.

Putative positive phage plaques identified by these means were picked, replated at low density (about 100 plaque forming units on each 9 cm diameter plate) and further screened for hybridisation to labelled oligonucleotide. The A.chrysogenum DNA portion from one example of a positive clone, λ 3/1, is illustrated in Figure 1(a). The map of this DNA is not exhaustive for all known restriction endonucleotide cleavage sites and is intended for illustrative purposes.

### Preparation 4: Construction of Plasmids pUX1 and pUX2

(a) Recovery of the 7 kb-BamHI restriction fragment of λ 3/1
   About 10 µg λ 3/1 DNA was digested with 25 units BamHI in a 50 µl reaction containing 20 mM Tris-HCl, 100 mM NaCl, 6 mM MgCl₂, 3 mM 2-mercaptoethanol at 37 °C for 2 hours. The digested DNA was resolved on a 0.7% agarose gel by electrophoresis under standard conditions. The 7 kb fragment was identified by reference to marker fragments of known size and excised from the gel. DNA was recovered by the method of Vogelstein and Gillespie (Proc.Natl.Acad.Sci.USA 76 (1979): 615 - 619) using a Geneclean Kit (BIO 101 Inc., La Jolla, CA 92038-2284, USA).
(b) Preparation of pUC18 Vector
   About 2 µg DNA of the widely available plasmid pUC18 (Yanisch-Perron et al, Gene 33 (1985): 103 - 109) was digested with 10 units BamHI at 37 °C in a 10 µl volume using conditions described in preparation 4(a). DNA was precipitated by addition of ammonium acetate to 2 M and 2 volumes of cold ethanol. The DNA pellet recovered by centrifugation was raised in 20 ul of 50 mM Tris-HCl pH 8.0, 0.1 mM EDTA and incubated with 0.5 units of Alkaline Phosphatase (Boehringer) at 37 °C for 1 hour. The phosphatase was inactivated by addition of EGTA to 10 mM and heating to 65 °C for 45 minutes. The DNA was precipitated by addition of sodium acetate to 0.3 M and 2 volumes of cold ethanol. The DNA pellet was raised in 20 µl TE buffer.
(c) Recovery and Identification of Clones
   Approximately 100 ng of dephosphorylated BamHI cut pUC18 DNA was ligated with 200 ng of isolated 7 kb-BamHI fragment DNA in a 10 µl ligation reaction using conditions described in preparation 2(b).
   The ligated DNA mixture was used to transform the widely available E.coli strain DH5α following the method of Chung and Miller (Nucl. Acids Res. 16 (1988): 3580). A 10 ml culture of bacterial cells grown in LB medium to optical density at 600 nm of 0.4 was harvested by centrifugation. The cells were resuspended in 1 ml LB medium (pH 6.1) containing 10% PEG 4000, 5% DMSO, 10 mM MgCl₂, 10 mM MgSO₄ (TSB) at 4 °C and incubated on ice for 10 - 30 minutes. For transformation, 0.1 ml aliquots of these cells were added to DNA ligation mixes or controls in cold 1.5 ml polypropylene tubes. The mixtures were incubated on ice for a further 30 minutes before the addition of 0.9 ml TSB containing 20 mM glucose. Tubes were transferred to a shaking incubator (37 °C, 200 rpm) and incubated for 60 minutes. The cell suspension was brought to 40 µg/ml in IPTG and plated onto L-agar containing 50 µg/ml ampicillin and 40 µg/ml X-gal, then incubated overnight at 37 °C. Colonies that contain only pUC18 plasmid appear blue under these conditions. Colonies that contain pUC18 with additional inserted DNA appear white. A number of white colonies were picked and plasmid DNA recovered by a modification of the alkaline SDS method of Birnboim and Doly (Maniatis et al, loc cit). The plasmids were screened by restriction analysis for the presence of the 7 kb -BamHI fragment. Two such plasmids, pUX1 and pUX2 contain this fragment in opposite orientations relative to the pUC18 vector portion of the construct. The A.chrysogenum DNA portion of pUX1 and pUX2 is depicted in Figure 1(b).

### Preparation 5: Construction of Plasmids pUX3 and pUX4

(a) Recovery of the 5.8 kb-EcoRI Fragment of λ 3/1
   About 10 µg λ 3/1 DNA was digested with 25 units EcoRI in a 50 µl reaction containing 100 mM Tris-HCl pH 7.5, 50 mM NaCl, 6 mM MgCl₂, 6 mM 2-mercaptoethanol at 37 °C for 2 hours. The digested DNA was resolved on a 0.8% agarose gel by electrophoresis under standard conditions. The 5.8 kb-EcoRI fragment was identified and recovered in substantial accordance with the procedure of preparation 4(a).
(b) Preparation of pUC18 Vector
   About 2 µg DNA of pUC18 was digested with 10 units EcoRI in a 10 µl reaction using conditions described in preparation 5(a). The linearised plasmid was further treated in substantial accordance with the procedure of preparation 4(b).
(c) Recovery and Identification of Clones
   Ligation of the 5.8 kb-EcoRI fragment to pUC18 and tranformation into E.coli strain DH5α were as described in preparation 4(c). The resultant plasmids were screened by restriction analysis for the presence of the 5.8 kb-EcoRI fragment. Two such plasmids, pUX3 and pUX4 contain this fragment in opposite orientations relative to the pUC18 vector portion of the construct. The A.chrysogenum portion of pUX3 and pUX4 is depicted in Figure 1(c) and also in Figure 2(a).

### Preparation 6: Construction of Plasmids pUX5, pUX6 and pUX11

(a) Recovery of BglII Fragments of λ 3/1
   About 10 µg λ 3/1 DNA was digested with 20 units BglII in a 50 µl reaction containing 10 mM Tris-HCl (pH 7.5), 7 mM MgCl₂, 100 mM NaCl, 7 mM 2-mercaptoethanol at 37 °C for 2 hours. The digested DNA was resolved on a 0.8% agarose gel by electrophoresis under standard conditions. The 5.2 kb- and 9.0 kb-fragments were identified and recovered in substantial accordance with the procedure of preparation 4(a).
(b) Preparation of pUC18 Vector
   About 2 µg of pUC18 DNA was prepared for use as cloning vector in accordance with the procedure of preparation 4(b).
(c) Recovery and Identification of Clones
   Aliquots containing about 200 ng of the isolated 5.2 kb- or 9.0 kb-Bg1II fragments of λ3/1 were each ligated to about 100 ng of dephosphorylated BamHI-cut pUC18 DNA in separate reactions according to the procedure of preparation 2(b). The resultant mixtures were transformed into E.coli DH5α cells according to the procedure of preparation 4(c). Recovered plasmids were screened by restriction analysis for the presence of inserted DNA. Plasmids pUX5 and pUX6 contain the 5.2 kb-BglII fragment in opposite orientations relative to the pUC18 portion of the construct. Plasmid pUX11 contains the 9.0 kb-BglII fragment. The A.chrysogenum DNA portions of plasmids pUX5 or pUX6 and pUX11 are illustrated in Figure 1(d) and (e) respectively.

### Preparation 7: Construction of Plasmids pUX7 and pUX8

(a) Recovery of the 4.5 kb-BamHI Fragment of pUX1
   About 5 µg of pUX1 DNA was digested with 25 units BamHI in a 50 µl reaction containing 20 mM Tris-HCl, pH 7.5, 100 mM NaCl, 6 mM MgCl₂, 3 mM 2-mercaptoethanol at 37 °C for 2 hours. The digested DNA fragments were resolved on a 0.7% agarose gel by electrophoresis. The 4.5 kb-fragment was identified and DNA recovered substantially in accordance with the procedure of preparation 4(a).
(b) Cloning of the DNA Fragment
   About 100 ng dephosphorylated BamHI-cut pUC18 DNA (preparation 4(b)) was ligated with 200 ng of isolated 4.5 kb-BamHI fragment and transformed into E.coli DH5α cells substantially in accordance with the procedure of preparation 4(c). Resultant transformant colonies were screened for the presence of recombinant plasmid DNA by restriction endonuclease analysis. Plasmids pUX7 and pUX8 were recognised as containing the A.chrysogenum 4.5 kb-BamHI fragment depicted in Figure 1(f). The plasmids pUX7 and pUX8 contain this fragment in opposite orientations relative to the pUC18 vector portion of the construct.

### Preparation 8: Construction of Plasmid pUX9

(a) Recovery of the 2.8 kb-SalI Fragment of pUX1
   About 5 µg of pUX1 DNA was digested with 20 units SalI in a 50 µl reaction containing 50 mM Tris.HCl (pH 7.5), 100 mM NaCl, 10 mM MgCl₂, 1 mM dithioerythritol at 37 °C for 2 hours. The 2.8 kb-fragment was resolved and recovered from a 1.0% agarose gel using substantially the procedure of preparation 4(a).
(b) Preparation of pUC18 Vector
   About 2 µg of pUC18 DNA was digested with 10 units SalI using the procedure of preparation 7(a), then dephosphorylated using the procedure of preparation 4(b).
(c) Recovery and Identification of Clones
   Aliquots of about 200 ng of the 2.8 kb SalI-fragment and 100 ng of dephosphorylated SalI-cut pUC18 were ligated according to the procedure of preparation 2(b). The resultant mixture was transformed into E.coli DH5α cells by the procedure of preparation 4(c). Recovered plasmids were screened by restriction analysis for the presence of inserted DNA. The plasmid pUX9 contains the 2.8 kb SalI fragment illustrated in Figure 2(b).

### Preparation 8: Characterisation of A.chrysogenum Strain ATCC 20371

Strain ATCC 20371 is obtainable from American Type Culture Collection. It is described as a DAC-overproducer and is therefore a good candidate for a strain defective in DAC acetyltransferase (DAT) enzyme activity.
(a) Growth of ATCC 20371
   Agar slope cultures of ATCC 20371 are prepared substantially in accordance with the procedure of preparation 1(b). Material is scraped from the surface of a well grown culture and inoculated into 10 ml seed medium in a 100 ml flask. A variety of proprietary media are applicable for this purpose. A suitable medium consists of 3% sucrose, 1.5% Lab Lemco (Difco), 0.5% corn steep liquor, 0.15% CaCO₃, pH 6.8. The culture is incubated for 3 days at 25.5 °C, 220 rpm. A 2.0 ml inoculum was then taken into 20 ml production medium in a 100 ml flask. A suitable medium consists of 3% sucrose, 3.2% soya bean flour, 0.5% DL-methionine, 0.15% CaCO₃, pH 6.8. This culture may be incubated for between 3 - 6 days at 25.5 °C, 220 rpm.
(b) Preparation of Cell-Free Extract
   Mycelium was harvested by vacuum filtration onto Whatman number 1 filter paper and washed with 100 ml ice cold 50 mM Tris-HCl pH 7.5. The mycelial pad was quick frozen in liquid nitrogen then ground to a fine powder in a pre-chilled mortar. The powder was resuspended in ice-cold 50 mM Tris-HCl, pH 7.5 at about 5 ml buffer per g mycelium. The suspension was then clarified by centrifugation at 15000 x g for 15 minutes at 4 °C. Supernatant fractions were used directly as crude extracts for assay of DAT activity.
(c) Assay of DAT Activity
   The DAT activity assay was based upon the conditions of Scheidegger et al (J Biotechnol. 3 (1985): 109 - 117) using DAC and freshly prepared acetyl-coenzyme A, each at 2 mM final concentrations. Assays were incubated for 30 - 120 minutes at 28 °C, 220 rpm. Reactions were stopped and protein precipitated by addition of 0.5 - 1 volume cold methanol. The clarified supernatants obtained on centrifugation were subjected to HPLC analysis to detect cephalosporin C formation.
   The strain C0728 (CMI 237183, obtainable from the C.A.B International Mycological Institute, Kew, Surrey TW9 3AF) was grown and protein extracts prepared in an identical manner to provide a positive control for DAT activity in these assays.
   In this manner it was possible to demonstrate DAC- and acetyl-CoA-dependent cephalosporin C synthesis by extracts of C0728 cells and to show that this activity was virtually absent (less than 1% of control level) in ATCC 20371 extracts.

### Preparation 9: Transformation of Strain ATCC 20371

(a) Growth of Cells and Preparation of Protoplasts
   Mycelium of strain ATCC 20371 was grown substantially in accordance with the procedure of preparation 1(b) and (c). The culture was incubated for 42 - 44 hours at 25.5 °C and 220 rpm. The mycelium was harvested by vacuum filtration onto Whatman number 1 filter paper and washed with 100 - 200 ml 1 M MgSO₄. Cells were resuspended in 1 M MgSO₄ containing 5 mg/ml Novozyme 234 (Novo Industri A/S, DK 2880 Bagvaerd, Denmark) and 10 MM DTT (components filter-sterilised). Sufficient buffer was added to allow about 1 g wet weight cells per 20 ml of buffer. The suspension, was incubated in a 250 ml flask at 25 °C and 220 rpm for about 90 - 120 minutes. Protoplast formation was monitored microscopically during this period. Protoplasts were recovered from the suspension by filtration through a glass sinter (porosity 2) under slight vacuum. Washing the sinter with a small volume of 1 M MgSO₄ improved recovery of protoplasts. The suspension was diluted with an equal volume of 0.8 M NaCl and protoplasts were harvested by centrifugation at 4000 g and 4 - 10 °C for 10 minutes in a bench top centrifuge. Protoplasts were resuspended in a total of 20 ml 0.5 M MgSO₄/0.4 M NaCl, washed and finally resuspended to a concentration of between 2 x 10⁸ to 1 x 10⁹ protoplasts per ml.
(b) Transformation Procedure
   DNA of λ3/1 or the plasmids of preparations 4, 5, 6, 7 or 8 were co-transformed into ATCC 20371 protoplasts along with plasmids of the type described by Kück et al (Appl. Microbiol. Biotechnol. 31 (1989): 358 - 365) which confer resistance to hygromycin B upon the host cell. For each mixture of plasmids to be transformed, a 250 µl aliquot of ATCC 20371 protoplasts was adjusted to 50 mM in CaCl₂. About 20 µg DNA was added in a 10 - 40 µl volume of TE buffer and the mixture incubated for 20 minutes at room temperature. A 250 µl aliquot of polyethylene glycol solution (50% w/v PEG-1000 (BDH Ltd, Broom Road, Poole, Dorset BH12 4NN) 50 mM Tris-HCl pH 7.5, 50 mM CaCl₂, 0.8 M NaCl; filter-sterilized) was added, mixed gently and incubation continued at room temperature for a further 20 minutes. The tube was then filled with 1 M MgSO₄ (approximately 1 ml), inverted several times to mix contents and centrifuged at 6500 rpm in an MSE Microcentaur centrifuge (MSE Scientific Instruments, Manor Royal, Crawley, Sussex RH10 2QQ) for 2 minutes. The protoplast pellet was resuspended in 500 µl 1 M MgSO₄ and 100 µl aliquots spread on the surface of stabilised agar plates. Stabilised agar contained 20% sucrose, 0.4% soluble starch, 1% bacto-yeast extract, 2% agar, pH 6.9 - 7.1. Plates were incubated for 16 - 24 hours at room temperature then overlayered with 6 ml of molten (42 °C) 0.4% Tryptone-Soya agar (Difco) containing 50 µg/ml hygromycin B (Boehringer Corporation London). After allowing the overlay to solidify, the plates were incubated at 25 °C for 12 - 14 days in a humidified atmosphere. Transformant colonies grew strongly through the overlay. The transformant colonies were subcultured to fresh agar containing 5 µg/ml hygromycin B.

### Preparation 10: Examination of Transformants

By these means a number of transformant colonies were derived from strain ATCC 20371 which were likely to contain copies of the DNA of preparations 3, 4, 5, 6, 7 or 8. These isolates were examined for the ability to synthesise cephalosporin C. Restoration of this ability in transformants of ATCC 20371 showed that the genetic defect in this strain that leads to loss of DAT activity has been complemented by activity of DAT encoded by the additional DNA transformed into the strain. Because of the nature of co-transformations, not all isolates recovered will be expected to carry copies of the non-selected DNA molecules. Thus, it is necessary to screen at least 50 - 100 transformants of each type to give an assessment of cephalosporin C biosynthesis.
(a) Bioassay Assessment
   The micro-organism Alcaligenes faecalis (ATCC 8750) is sensitive to cephalosporin C in the µg/ml dilution range but resistant to mg/ml concentration of DAC. It can be used as a sensitive indicator organism for cephalosporin C in the presence of DAC. The means of conducting such bioassays are well known in the art.
   Purified isolates derived from transformation experiments following the procedure of preparation 9 were grown on agar plates in the presence of 5 µg/ml hygromycin for 5 or 6 days at 25.5 °C in a humidified atmosphere. Agar plugs were cut from the confluent growth areas of such plates using a sterile number 4 size cork borer. These plugs were manipulated onto the surface of seeded bioassay dishes. The level of hygromycin B present in these plugs was insufficient to cause any inhibition of A.faecalis growth. Bioassay dishes were prepared by the following means.
   A culture of A.faecalis was grown in L-broth overnight at 37 °C. A secondary log phase culture was developed by subculture (2 ml culture to 50 ml fresh L-broth) and growth at 37 °C with shaking at 250 rpm for 3 hours. This culture was seeded into molten (42 °C) agar at about 1 ml culture per 100 ml agar. A 0.25 ml aliquot of 0.5% 2, 3, 5 triphenyltetrazolium chloride (Tetrazolium Red, Sigma Chemical Co) was also added per 100 ml agar before preparing agar plates.
   The prepared plates were incubated overnight at 37 °C to allow growth of the β-lactam sensitive test organism. Under these conditions the ATCC 20371 strain produces little or no inhibition zone in the A.faecalis growth. Various isolates from transformation of this strain using λ3/1, pUX1, pUX2, pUX3, pUX4, pUX9 or pUX11 DNAs showed greatly increased inhibition zones indicating the production of cephalosporin C. In contrast, tranformants obtained using pUX5, pUX6, pUX7 or pUX8 DNAs showed no cephalosporin C production.
   The production of cephalosporin C by isolates was confirmed by analysis of broth samples from additional fermentations of the selected isolates. These fermentations were carried out substantially in accordance with the procedure of preparation 8(a). Broth samples (1 ml) were taken at intervals between 3 - 6 days and clarified by centrifugation. The supernatants were mixed with 0.5 - 1 volume methanol, allowed to stand at room temperature for 30 - 60 minutes then further clarified by centrifugation. Samples were analysed by HPLC in accordance with the procedure described in preparation 8(c). By these means the presence of substantial quantities of cephalosporin C was detected in samples of selected isolate fermentations. The same isolates were further examined for the presence of DAT enzyme activity substantially by the procedure described in preparation 8 (b) and (c). All those isolates showing increased bioassay response and cephalosporin C production also showed DAT activity by these means. In addition, selected isolates were confirmed as transformants by demonstration of the presence of DNA of the transforming plasmids integrated into the host genome. This was achieved by hybridisation of transformant DNA prepared by the procedure of preparation 1(c) and (d) with ³²P-labelled DNA of the transforming vectors using the standard Southern blot methodology essentially as described (Maniatis et al., loc cit.).
   By these means the region of A.chrysogenum DNA encoding DAT has been localised to the area indicated in Figure 2(e).

### Preparation 11: Construction of M13 Subclone K18/4

(a) Recovery of the 1.2 kb-KpnI Fragment of pUX3
   About 5 µg pUX3 DNA was digested with 20 units KpnI in a 25 µl reaction containing 6 mM Tris.HCl (pH 7.5), 6 mM NaCl, 6 mM MgCl₂ and 6 mM 2 mercaptoethanol at 37 °C for 2 hours. The 1.2 kb-KpnI fragment was resolved and recovered from a 1.0% agarose gel using substantially the procedure of preparation 4(a).
(b) Preparation of M13mp18 Vector
   About 2 µg DNA of the widely available bacteriophage vector M13mp18 was digested with 10 units KpnI under the conditions of preparation 11(a).
(c) Recovery and Identification of Clones
   Aliquots containing about 100 ng of the isolated 1.2 kb-KpnI fragment and 50 ng of the KpnI-cut M13mp18 DNA were ligated substantially according to the method of preparation 4(c). The resultant DNA mixture was transformed into the widely available E.coli host strain TG1. A culture of TG1 was grown overnight at 37 °C in 2 x TY broth (2 x TY broth contains 1.6% Bacto-tryptone, 1% yeast extract, 0.5% NaCl). A 2 ml aliquot of this culture was inoculated into 40 ml fresh pre-warmed 2 x TY broth in a 250 ml flask and grown with shaking (250 rpm) at 37 °C until the cultured reached an absorbance (600 nm) of about 0.4. Cells were harvested by centrifugation (4000 rpm for 5 minutes in a refrigerated bench top centrifuge and resuspended in 20 ml ice cold 50 mM CaCl₂. The suspension was incubated on ice for 20 minutes, then the cell were harvested by centrifugation. The cell pellet was resuspended in 4 ml ice cold 50 mM CaCl₂. Aliquots of treated cells (0.3 ml) were added to the ligation mixtures or controls and incubated on ice for 40 - 60 minutes. Tubes were then transferred to a water bath at 42 °C for 3 minutes and returned to ice temperature. To each transformation mix was then added a 5 ml aliquot of molten (42 °C) H top agar containing final concentrations of 1 mM IPTG and 200 µg/ml XGal. H top agar consists of 1% Bacto-tryptone, 0.8% NaCl, 0.8% agar.
   After mixing, the contents were poured immediately onto fresh L-agar plates and allowed to solidify. Plates were incubated overnight at 37 °C. Plaques of the parental M13mp18 type appear blue under these conditions. Recombinant types appear colourless. A number of recombinant types were picked and the bacteriophage particles grown using standard conditions well known in the art (see for example Perbal B: A Practical Guide to Molecular Cloning (Second Edition), Wiley Interscience, New York, 1988).
   Individual isolates were screened for the presence of the 1.2 kb-KpnI fragment by restriction analysis. One such isolate, termed M13K18/4 was deposited under the terms of the Budapest Treaty on the International Recognition of the Deposit of Micro-organisms for the Purposes of Patent Procedure (1977), at the National Collection of Industrial and Marine Bacteria, 135 Abbey Road, Aberdeen AB9 8DG, on 15 December 1989 under the Accession Number NCIMB 40236. The A.chrysogenum DNA portion of this construct is illustrated in Figure 2(c).
   M13K18/4 may be used to identify DAT-encoding DNA from any A.chrysogenum strain or genomic library obtained from A.chrysogenum DNA or RNA. Single stranded DNA of M13K18/4 is prepared by standard means well known in the art (Perbal, loc cit). The single stranded DNA is labelled with ³²P- or ³⁵S- radionucleotides by DNA polymerase enzyme action, conveniently achieved using a Multiprime™ DNA labelling system (Amersham International plc). Alternatively the double stranded DNA form of M13K18/4 may be purified by standard means (Maniatis et al, loc cit, Perbal, loc cit) and the 1.2 kb-KpnI fragment recovered following the procedure of preparation 4(a). This double stranded DNA or the derived fragment may then be labelled by standard means for use as alternative hybridisation probes.
   Alternative plasmids or DNA fragments including portions of the DAT-encoding region may also be utilised as hybridisation probes. Suitable probes would include the A.chrysogenum DNA depicted in Figure 1(b), derived from pUX1 or pUX2; the DNA depicted in Figure 1(c), derived from pUX3 or pUX4; the DNA depicted in Figure 2(b), derived from pUX9; or alternatively the DNA fragments depicted in Figure 2(d) or Figure 2(e).

### Preparation 12: A.chrysogenum Directed Genomic Library Construction

(a) Preparation of DNA Fragments
   A 250 µg aliquot of A.chrysogenum (ATCC 14553) DNA prepared in substantial accordance with the procedure of preparation 1, was mixed with 100 µl 10 x One-Phor-All Plus™ buffer (Pharmacia-LKB Biotechnology, S751 82 Uppsala, Sweden) and adjusted to a final volume of 1000 µl including 100 units BamHI and 100 units XbaI restriction enzymes. The mixture was incubated for 2 hours at 37 °C. The resultant DNA fragments were resolved by centrifugation through a 10 - 40% w/v sucrose gradient in accordance with the procedure of preparation 2(a). The gradient was collected as 0.5 ml fractions and the DNA of size range 5 - 8 kb (as determined by agarose gel electrophoresis) was pooled, adjusted to 0.3 M in sodium acetate and precipitated by addition of 2 volumes ice cold ethanol. The DNA pellet recovered by centrifugation was raised in 25 µl TE buffer.
(b) Preparation of pSU20 Vector
   About 5 µg DNA of the plasmid pSU20 was digested with 10 units BamHI and 10 units XbaI in 1 x One-Phor-All Plus buffer for 1 hour at 37 °C. Plasmid pSU20 was obtained from Dr F de la Cruz (Departmento de Biologio Molecular, Universidad de Cantabria, 39001 Santander, Spain) and is derived from pSU2713 (Martinez et al Gene 68 (1988): 159-162). The digested DNA was precipitated by addition of EDTA to 20 mM, ammonium acetate to 2M and 2 volumes cold ethanol. The precipitate was recovered by centrifugation, raised in 100 µl TE buffer and subjected to a further ammonium acetate/ethanol precipitation. The final DNA pellet was raised in 10 µl TE buffer.
(c) Preparation of Directed Genomic Library
   The A.chrysogenum DNA BamHI - XbaI fragments of 5 -8 kb size range were mixed with BamHI - XbaI cut pSU20 DNA, using around 100 ng and 50 ng aliquots of each respectively. The DNA mixture was ligated using T4 DNA ligase substantially in accordance with the procedure of preparation 2(b). The ligation mix was used to transform E.coli DH5α cells substantially in accordance with the procedure of preparation 4(c), except that the agar plates used at the final stage contained 30 µg/ml chloramphenicol. The resultant transformed colonies represent a directed library of A.chrysogenum DNA cloned as fragments of between 5 - 8 kb in size with ends defined by BamHI and XbaI restriction sites.

### Preparation 13: Isolation of Clones from the Directed Plasmid Library

(a) Preparation of Hybridisation Probe
   About 5 µg DNA of the plasmid pUX2 was digested with 20 units HindIII in a 25 µl reaction containing 10 mM Tris-HCl pH 7.5, 50 mM NaCl, 10 mM MgCl₂, 10 mM dithiothreitol at 37 °C for 2 hours. The digested DNA fragments were resolved on a 0.8% agarose gel by electrophoresis under standard conditions. The 2.0 kb-fragment (Figure 2(d)) was identified and DNA recovered substantially in accordance with the procedure of preparation 4.
(b) Screening of Directed Library Isolates
   About 5000 transformant colonies of E.coli strain DH5α obtained in preparation 12 were grown on a 20 x 20 cm agar plate. Duplicate nylon membrane (Hybond-N, Amersham International plc) replicates were prepared from this plate using standard methods as outlined in preparation 3. The membranes were incubated in 50 ml prehybridisation buffer in accordance with the procedure of preparation 3.
   About 25 ng DNA of the isolated 2.0 kb HindIII fragment of pUX2, obtained as described in preparation 13(a) were radioactively labelled with ³²P-dATP using a Multiprime™ DNA labelling system (Amersham International plc), following the manufacturer's guidelines. The labelled DNA was mixed with 50 ml fresh pre-hybridisation buffer, then incubated with the prepared membranes at 65 °C overnight. The membranes were washed free of excess label by incubation in 2 changes of about 200 ml 3 x SSC, 0.1% SDS buffer each for 20 minutes at room temperature, followed by 2 changes of this buffer each for 20 minutes duration at 65 °C. Damp-dry membranes were exposed to X-ray photographic film (Hyperfilm-MP, Amersham International plc) in the presence of intensifying screens at -20 °C for 24 - 48 hours.
   By these means 6 colonies showed positive hybridisation to the labelled probe, indicating the presence of homologous DNA sequences. Plasmid DNA was recovered from these isolates and subjected to restriction analysis to confirm the presence of the 6.0 kb BamHI - XbaI fragment indicated in Figure 1(g). One of these plasmids was designated pSG1.

### Preparation 14: Construction of Plasmid pSG2

(a) Recovery of the 4.4 kb-BamHI - BglII Fragment of pSG1
   About 5 µg of pSG1 DNA was digested with 8 units BglII in a 50 µl reaction containing 10 mM Tris-HCl, pH 7.5, 50 mM NaCl, 10 mM MgCl₂, 1 mM Dithiothreitol for 2 hours at 37 °C. The reaction mix was adjusted to 100 mM NaCl, 12 units BamHI and 12.5 units EcoRI were added and the incubation continued for a further hour. The digested DNA was resolved on a 0.8% agarose gel and the 4.4 kb-fragment identified, excised and DNA recovered in accordance with the procedure of preparation 4(a).
(b) Preparation of pSU20 Vector
   About 2 µg DNA of plasmid pSU20 was digested with 10 units BamHI and treated with alkaline phosphatase substantially in accordance with the procedure of preparation 4(b) except that inactivation of phosphatase activity was achieved by incubation of the reaction mix at 85 °C for 15 minutes.
(c) Recovery and Identification of Clones
   About 100 ng of the dephosphorylated BamHI-cut pSU20 was ligated with about 200 ng of the isolated 4.4 kb-BamHI BglII fragment following the procedure of preparation 2(b). The ligated DNA mix was used to transform E.coli DH5α following the procedure of preparation 4(c). Resultant transformant colonies were screened for the presence of recombinant plasmid DNA by restriction analysis.
   By these means the plasmid pSG2 was identified and shown to contain the 4.4 kb-BamHI- BglII fragment inserted into the pSU20 vector molecule. Plasmid pSG2 was deposited under the terms of the Budapest Treaty at the National Collection of Industrial and Marine Bacteria, Aberdeen, on the 27 February 1990 under the Accession Number NCIMB 40264. The A.chrysogenum portion of this construct is illustrated in Figure 2(e).

As referenced in the accompanying text, the following drawings are included:
Figure 1
   (a) A restriction map of the A.chrysogenum DNA portion of the clone λ3/1.
   (b) A restriction map of the A.chrysogenum DNA portion of clones pUX1 and pUX2.
   (c) A restriction map of the A.chrysogenum DNA portion of clones pUX3 and pUX4.
   (d) A restriction map of the A.chrysogenum DNA portion of clones pUX5 and pUX6.
   (e) A restriction map of the A.chrysogenum DNA portion of clone pUX11.
   (f) A restriction map of the A.chrysogenum DNA portion of clones pUX7 and pUX8.
   (g) A restriction map of the A.chrysogenum DNA portion of clone pSG1.
Figure 2
   (a) An enlarged restriction map of the A.chrysogenum DNA portion of clones pUX3 and pUX4.
   (b) A restriction map of the A.chrysogenum DNA portion of clone pUX9.
   (c) A restriction map of the A.chrysogenum DNA portion of clone M13K18/4.
   (d) A restriction map of a 2 kb-HindIII fragment of A.chrysogenum DNA.
   (e) A restriction map of the A.chrysogenum DNA portion of clone pSG2.

SEQ ID NO: 1
SEQUENCE TYPE: Nucleotide with corresponding peptide
SEQUENCE LENGTH : 459 nucleotides
STRANDEDNESS: single
TOPOLOGY: linear
MOLECULAR TYPE: genomic DNA
ORIGINAL SOURCE ORGANISME: Acremonium chrysogenum (ATCC 14553)
EXPERMENTAL SOURCE: Plasmid pSG2 (NCIMB 40264)
FEATURES: sequence begins 95 nucleotides from BglII site of pSG2
PROPERTIES: amino terminal sequence cefG gene

ATG CTG CCG TCG GCC CAA GTG GCC CGT CTA AAG CCG GAC CCC TTT CCC 48
Met Leu Pro Ser Ala Gln Val Ala Arg Leu Lys Pro Asp Pro Phe Pro
                5                   10                  15

CCG AGT CTC TCC CCG ATC CCG CAC GGG GCC GTC ACT TTC GCT GCC CTC 96
Pro Ser Leu Ser Pro Ile Pro His Gly Ala Val Thr Phe Ala Ala Leu
            20                  25                  30

GCT CCT TGT CAT AAC CTA CCT ATA TTC TCA TCC CGG CAA ATG CTG CGG 144
Ala Pro Cys His Asn Leu Pro Ile Phe Ser Ser Arg Gln Met Leu Arg
        35                  40                  45

GAT AGC CTC ACC TAC AGC CAC ACG TCG CCC ACC ATG TCG CCT CAG ATC 192
Asp Ser Leu Thr Tyr Ser His Thr Ser Pro Thr Met Ser Pro Gln Ile
    50                  55                  60

GCC AAT CGC TTC GAG GCT TCG CTA GAT GCC CAA GAC ATA GCC AGA ATA 240
Ala Asn Arg Phe Glu Ala Ser Leu Asp Ala Gln Asp Ile Ala Arg Ile
65                  70                  75                  80

TCG CTC TTC ACA CTG GAA TCT GGC GTC ATC CTT CGC GAT GTA CCC GTG 288
Ser Leu Phe Thr Leu Glu Ser Gly Val Ile Leu Arg Asp Val Pro Val
                85                  90                  95

GCA TAC AAA TCG TGG GGT CGC ATG AAT GTC TCA AGG GAT AAC TGC GTC 336
Ala Tyr Lys Ser Trp Gly Arg Met Asn Val Ser Arg Asp Asn Cys Val
            100                 105                 110

ATC GTC TGC CAC ACC TTG ACG AGC AGC GCC CAT GTC ACC TCG TGG TGG 384
Ile Val Cys His Thr Leu Thr Ser Ser Ala His Val Thr Ser Trp Trp
        115                 120                 125

CCC ACA CTG TTT GGC CAA GGC AGG GCT TTC GAT ACC TCT CGC TAC TTC 432
Pro Thr Leu Phe Gly Gln Gly Arg Ala Phe Asp Thr Ser Arg Tyr Phe
    130                 135                 140

ATC ATC TGC CTA AAT TAT CTC GGG AGC
Ile Ile Cys Leu Asn Tyr Leu Gly Ser
145                 150

## Claims (Claims for the following Contracting State(s): AT, BE, CH, DE, DK, FR, GB, IT, LI, LU, NL, SE)

1. A recombinant DNA sequence or a restriction fragment thereof containing a DAC acetyltransferase gene obtainable from Acremonium chrysogenum, encoding a protein comprising the amino terminal structure of 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 but not containing a gene coding on expression for the native DAOCS/DACS polypeptide.

2. A recombinant DNA sequence or a restriction fragment thereof containing the DAC acetyltransferase gene obtainable from Acremonium chrysogenum, encoding a protein comprising the amino terminal structure of 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 but not containing the gene coding on expression for DAOCS/DACS activity.

3. A recombinant DNA sequence as claimed in any either of Claim 1 or 2 wherein DAC acetyl transferase comprises the nucleotide sequence of

5'-ATG CTG CCG TCG GCC CAA GTG GCC CGT CTA AAG CCG GAC CCC TTT CCC
CCG AGT CTC TCC CCG ATC CCG CAC GGG GCC GTC ACT TTC GCT GCC CTC
GCT CCT TGT CAT AAC CTA CCT ATA TTC TCA TCC CGG CAA ATG CTG CGG
GAT AGC CTC ACC TAC AGC CAC ACG TCG CCC ACC ATG TCG CCT CAG ATC
GCC AAT CGC TTC GAG GCT TCG CTA GAT GCC CAA GAC ATA GCC AGA ATA
TCG CTC TTC ACA CTG GAA TCT GGC GTC ATC CTT CGC GAT GTA CCC GTG
GCA TAC AAA TCG TGG GGT CGC ATG AAT GTC TCA AGG GAT AAC TGC GTC
ATC GTC TGC CAC ACC TTG ACG AGC AGC GCC CAT GTC ACC TCG TGG TGG
CCC ACA CTG TTT GGC CAA GGC AGG GCT TTC GAT ACC TCT CGC TAC TTC
ATC ATC TGC CTA AAT TAT CTC GGG AGC-3'

4. A recombinant DNA expression vector comprising a recombinant DNA sequence as claimed in either one of Claims 1 to 3.

5. A recombinant DNA expression vector comprising a recombinant DNA sequence as claimed in either one of Claims 1 to 3 and additionally comprising a promoter and translation activating sequence operatively linked to the DAC acetyltransferase-encoding DNA sequence.

6. A recombinant DNA expression vector as claimed in Claim 5 wherein said promoter and translation activating sequence are isolated from the same species as that from which the DAC acetyltransferase-encoding DNA sequence is isolated.

7. A eukaryotic or prokaryotic host transformed with a recombinant DNA expression vector as claimed in any one of Claims 4 to 6.

8. A prokaryotic host as claimed in Claim 7 selected from a species of filamentous fungi, a species of streptomyces or a bacteria.

9. A host as claimed in Claim 8 selected from Acremonium spp., Penicillium spp., E.coli or Bacillus spp.

10. A host as claimed in Claim 9 selected from Acremonium chrysogenum or Penicillium chrysogenum.

11. A method of expressing DAC acetyltransferase activity in a eukaryotic or prokaryotic host comprising: -
a) transforming the host cell with a recombinant DNA expression vector that comprises (i) and expression control sequence that functions in the host cell, and (ii) a DAC acetyltransferase-encoding DNA sequence according to claim 1 or 2 which is operatively linked to the expression control sequence; and
b) culturing the host cell transformed in step (a) under conditions that allow for expression of DAC acetyltransferase activity.

12. A method as claimed in Claim 11 wherein the host cell is selected from Acremonium spp., Penicillium spp., E.coli or Basillus spp.

13. A method as claimed in Claim 12 wherein the host cell is selected from Acremonium chrysogenum or Penicillium chrysogenum.

## Claims (Claims for the following Contracting State(s): ES, GR)

1. A method for preparing a recombinant DNA expression vector, comprising ligating together a recombinant DNA sequence or a restriction fragment thereof containing a gene coding on expression for DAC acetyltransferase activity obtainable from Acremonium chrysogenum, said recombinant DNA sequence encodes a protein comprising the amino terminal structure of 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 but not containing a gene coding on expression for the native DAOCS/DACS polypeptide, and a DNA sequence that encodes a promoter and translation activating sequence operatively linked to the DAC acetyltransferase-encoding DNA sequence.

2. A method for preparing a recombinant DNA expression vector, comprising ligating together a recombinant DNA sequence or a restriction fragment thereof containing the gene coding on expression for DAC acetyltransferase activity obtainable from Acremonium chrysogenum, said recombinant DNA sequence encodes a protein comprising the amino terminal structure of 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 but not containing the gene coding on expression for DAOCS/DACS activity, and a DNA sequence that encodes a promoter and translation activating sequence operatively linked to the DAC acetyltransferase-encoding DNA sequence.

3. A method as claimed in Claims 1 or 2 wherein said promoter and translation activating sequence are isolated from the same species as that from which the DAC acetyltransferase-encoding DNA sequence is isolated.

4. A method as claimed in any one of claims 1 to 3, wherein said recombinant DNA sequence comprises the nucleotide sequence of

5'-ATG CTG CCG TCG GCC CAA GTG GCC CGT CTA AAG CCG GAC CCC TTT CCC
CCG AGT CTC TCC CCG ATC CCG CAC GGG GCC GTC ACT TTC GCT GCC CTC
GCT CCT TGT CAT AAC CTA CCT ATA TTC TCA TCC CGG CAA ATG CTG CGG
GAT AGC CTC ACC TAC AGC CAC ACG TCG CCC ACC ATG TCG CCT CAG ATC
GCC AAT CGC TTC GAG GCT TCG CTA GAT GCC CAA GAC ATA GCC AGA ATA
TCG CTC TTC ACA CTG GAA TCT GGC GTC ATC CTT CGC GAT GTA CCC GTG
GCA TAC AAA TCG TGG GGT CGC ATG AAT GTC TCA AGG GAT AAC TGC GTC
ATC GTC TGC CAC ACC TTG ACG AGC AGC GCC CAT GTC ACC TCG TGG TGG
CCC ACA CTG TTT GGC CAA GGC AGG GCT TTC GAT ACC TCT CGC TAC TTC
ATC ATC TGC CTA AAT TAT CTC GGG AGC-3'

5. A method of expressing DAC acetyltransferase activity in a eukaryotic or prokaryotic host comprising:
a) transforming the host cell with a recombinant DNA expression vector that comprises (i) and expression control sequence that functions in the host cell, and (ii) a DAC acetyltransferase-encoding DNA sequence according to claim 1 or 2 which is operatively linked to the expression control sequence; and
b) culturing the host cell transformed in step (a) under conditions that allow for expression of DAC acetyltransferase activity.

6. A method as claimed in Claim 5 wherein the host cell is selected from Acremonium spp., Penicillium spp., E.coli or Bacillus spp.

7. A method as claimed in Claim 6 wherein the host cell is selected from Acremonium chrysogenum or Penicillium chrysogenum.

## Patentansprüche (Patentansprüche für folgende(n) Vertragsstaat(en): AT, BE, CH, DE, DK, FR, GB, IT, LI, LU, NL, SE)

1. Rekombinante DNA-Sequenz oder ein Restriktionsfragment davon, enthaltend ein DAC-Acetyltransferase-Gen, erhältlich aus Acremonium chrysogenum, das ein Protein kodiert, umfassend die aminoterminale Struktur: 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 aber nicht ein Gen enthaltend, das bei Expression für das native DAOCS/DACS-Polypeptid kodiert.

2. Rekombinante DNA-Sequenz oder ein Restriktionsfragment davon, enthaltend das DAC-Acetyltransferase-Gen, erhältlich aus Acremonium chrysogenum, das ein Protein kodiert, umfassend die aminoterminale Struktur: 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 aber nicht das Gen enthaltend, das bei Expression tür DAOCS/DACS-Aktivität kodiert.

3. Rekombinante DNA-Sequenz gemäß Anspruch 1 oder 2, worin DAC-Acetyltransferase die Nukleotidsequenz 
5'-ATG CTG CCG TCG GCC CAA GTG GCC CGT CTA AAG CCG GAC CCC TTT CCC
CCG AGT CTC TCC CCG ATC CCG CAC GGG GCC GTC ACT TTC GCT GCC CTC
GCT CCT TGT CAT AAC CTA CCT ATA TTC TCA TCC CGG CAA ATG CTG CGG
GAT AGC CTC ACC TAC AGC CAC ACG TCG CCC ACC ATG TCG CCT CAG ATC
GCC AAT CGC TTC GAG GCT TCG CTA GAT GCC CAA GAC ATA GCC AGA ATA
TCG CTC TTC ACA CTG GAA TCT GGC GTC ATC CTT CGC GAT GTA CCC GTG
GCA TAC AAA TCG TGG GGT CGC ATG AAT GTC TCA AGG GAT AAC TGC GTC
ATC GTC TGC CAC ACC TTG ACG AGC AGC GCC CAT GTC ACC TCG TGG TGG
CCC ACA CTG TTT GGC CAA GGC AGG GCT TTC GAT ACC TCT CGC TAC TTC
ATC ATC TGC CTA AAT TAT CTC GGG AGC-3'
 umfaßt.

4. Rekombinanter DNA-Expressionsvektor, umfassend eine rekombinante DNA-Sequenz gemäß einem der Ansprüche 1 bis 3.

5. Rekombinanter DNA-Expressionsvektor, umfassend eine rekombinante DNA-Sequenz gemäß einem der Ansprüche 1 bis 3 und zusätzlich umfassend eine Promotor- und translationsaktivierende Sequenz, die funktionsfähig mit der DAC-Acetyltransferase-kodierenden DNA-Sequenz verknüpft sind.

6. Rekombinanter DNA-Expressionsvektor gemäß Anspruch 5, worin die Promotor- und translationsaktivierende Sequenz aus der gleichen Art isoliert sind wie die, aus der die DAC-Acetyltransferase-kodierende DNA-Sequenz isoliert wird.

7. Eukaryotischer oder prokaryotischer Wirt, transformiert mit einem rekombinanten DNA-Expressionsvektor gemäß einem der Ansprüche 4 bis 6.

8. Prokaryotischer Wirt gemäß Anspruch 7, ausgewählt aus einer Art aus fadenförmigen Pilzen, einer Art aus Streptomyces oder einem Bakterium.

9. Wirt gemäß Anspruch 8, ausgewählt aus Acremonium spp., Penicillium spp., E. coli oder Bacillus spp.

10. Wirt gemäß Anspruch 9, ausgewählt aus Acremonium chrysogenum oder Penicillium chrysogenum.

11. Verfahren zur Expression von DAC-Acetyltransferaseaktivität in einem eukaryotischen oder prokaryotischen Wirt, umfassend:
a) Transformieren der Wirtszelle mit einem rekombinanten DNA-Expressionsvektor, der umfaßt: (i) eine Expressionskontrollsequenz, die in der Wirtszelle arbeitet, und (ii) eine DAC-Acetyltransferase-kodierende DNA-Sequenz gemäß Anspruch 1 oder 2, die funktionsfähig mit der Expressionskontrollsequenz verknüpft ist; und
b) Kultivieren der in Schritt (a) transformierten Wirtszelle unter Bedingungen, die eine Expression von DAC-Acetyltransferaseaktivität erlauben.

12. Verfahren gemäß Anspruch 11, worin die Wirtszelle ausgewählt ist aus Acremonium spp., Penicillium spp., E. coli oder Bacillus spp.

13. Verfahren gemäß Anspruch 12, worin die Wirtszelle ausgewählt ist aus Acremonium chrysogenum oder Penicillium chrysogenum.

## Patentansprüche (Patentansprüche für folgende(n) Vertragsstaat(en): ES, GR)

1. Verfahren zur Herstellung eines rekombinanten DNA-Expressionsvektors, umfassend das Ligieren einer rekombinanten DNA-Sequenz oder eines Restriktionsfragments davon, enthaltend ein Gen, das bei Expression für DAC-Acetyltransferaseaktivität kodiert, erhältlich aus Acremonium chrysogenum, wobei die rekombinante DNA-Sequenz ein Protein kodiert, umfassend die aminoterminale Struktur: 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 aber nicht ein Gen enthaltend, das bei Expression für das native DAOCS/DACS-Polypeptid kodiert, zusammen mit einer DNA-Sequenz, die eine Promotor- und translationsaktivierende Sequenz kodiert, die funktionsfähig mit der DAC-Acetyltransferase-kodierenden DNA-Sequenz verknüpft sind.

2. Verfahren zur Herstellung eines rekombinanten DNA-Expressionsvektors, umfassend das Ligieren einer rekombinanten DNA-Sequenz oder eines Restriktionsfragments davon, enthaltend das Gen, das bei Expression für DAC-Acetyltransferaseaktivität kodiert, erhältlich aus aus Acremonium chrysogenum, wobei die rekombinante DNA-Sequenz ein Protein kodiert, umfassend die aminoterminale Struktur: 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 aber nicht das Gen enthaltend, das bei Expression für DAOCS/DACS-Aktivität kodiert, zusammen mit einer DNA-Sequenz, die eine Promotor- und translationsaktivierende Sequenz kodiert, die funktionsfähig mit der DAC-Acetyltransferase-kodierenden DNA-Sequenz verknüpft sind.

3. Verfahren gemäß Anspruch 1 oder 2, worin die Promotor- und translationsaktivierende Sequenz aus der gleichen Art isoliert sind wie die, aus der die DAC-Acetyltransferase-kodierende DNA-Sequenz isoliert wird.

4. Verfahren gemäß einem der Ansprüche 1 bis 3, worin die rekombinante DNA-Sequenz die Nukleotidsequenz 
5'-ATG CTG CCG TCG GCC CAA GTG GCC CGT CTA AAG CCG GAC CCC TTT CCC
CCG AGT CTC TCC CCG ATC CCG CAC GGG GCC GTC ACT TTC GCT GCC CTC
GCT CCT TGT CAT AAC CTA CCT ATA TTC TCA TCC CGG CAA ATG CTG CGG
GAT AGC CTC ACC TAC AGC CAC ACG TCG CCC ACC ATG TCG CCT CAG ATC
GCC AAT CGC TTC GAG GCT TCG CTA GAT GCC CAA GAC ATA GCC AGA ATA
TCG CTC TTC ACA CTG GAA TCT GGC GTC ATC CTT CGC GAT GTA CCC GTG
GCA TAC AAA TCG TGG GGT CGC ATG AAT GTC TCA AGG GAT AAC TGC GTC
ATC GTC TGC CAC ACC TTG ACG AGC AGC GCC CAT GTC ACC TCG TGG TGG
CCC ACA CTG TTT GGC CAA GGC AGG GCT TTC GAT ACC TCT CGC TAC TTC
ATC ATC TGC CTA AAT TAT CTC GGG AGC-3'
 umfaßt.

5. Verfahren zur Expression von DAC-Acetyltransferaseaktivität in einem eukaryotischen oder prokaryotischen Wirt, umfassend:
a) Transformieren der Wirtszelle mit einem rekombinanten DNA-Expressionsvektor, der umfaßt: (i) eine Expressionskontrollsequenz, die in der Wirtszelle arbeitet, und (ii) eine DAC-Acetyltransferase-kodierende DNA-Sequenz gemäß Anspruch 1 oder 2, die funktionsfähig mit der Expressionskontrollsequenz verknüpft ist; und
b) Kultivieren der in Schritt (a) transformierten Wirtszelle unter Bedingungen, die eine Expression von DAC-Acetyltransferaseaktivität erlauben.

6. Verfahren gemäß Anspruch 5, worin die Wirtszelle ausgewählt ist aus Acremonium spp., Penicillium spp., E. coli oder Bacillus spp.

7. Verfahren gemäß Anspruch 6, worin die Wirtszelle ausgewählt ist aus Acremonium chrysogenum oder Penicillium chrysogenum.

## Revendications (Revendications pour l'(les) Etat(s) contractant(s) suivant(s): AT, BE, CH, DE, DK, FR, GB, IT, LI, LU, NL, SE)

1. Séquence d'ADN recombinant ou fragment de restriction de celle-ci contenant un gène de DAC acétyltransférase obtenable d'Acremonium chrysogenum, codant pour une protéine comprenant la structure amino terminale de : 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 mais ne contenant pas de gène codant lors de l'expression pour le polypeptide de DAOCS/DACS natif.

2. Séquence d'ADN recombinant ou fragment de restriction de celle-ci contenant le gène de DAC acétytransférase obtenable d'Acremonium chrysogenum, codant pour une protéine comprenant la structure amino terminale de : 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 mais ne contenant pas le gène codant lors de l'expression pour une activité de DAOCS/DACS.

3. Séquence d'ADN recombinant suivant l'une ou l'autre des revendications 1 et 2, dans laquelle la DAC acétyltransférase comprend la séquence nucléotidique de :

5'-ATG CTG CCG TCG GCC CAA GTG GCC CGT CTA AAG CCG GAC CCC TTT CCC
CCG AGT CTC TCC CCG ATC CCG CAC GGG GCC GTC ACT TTC GCT GCC CTC
GCT CCT TGT CAT AAC CTA CCT ATA TTC TCA TCC CGG CAA ATG CTG CGG
GAT AGC CTC ACC TAC AGC CAC ACG TCG CCC ACC ATG TCG CCT CAG ATC
GCC AAT CGC TTC GAG GCT TCG CTA GAT GCC CAA GAC ATA GCC AGA ATA
TCG CTC TTC ACA CTG GAA TCT GGC GTC ATC CTT CGC GAT GTA CCC GTG
GCA TAC AAA TCG TGG GGT CGC ATG AAT GTC TCA AGG GAT AAC TGC GTC
ATC GTC TGC CAC ACC TTG ACG AGC AGC GCC CAT GTC ACC TCG TGG TGG
CCC ACA CTG TTT GGC CAA GGC AGG GCT TTC GAT ACC TCT CGC TAC TTC
ATC ATC TGC CTA AAT TAT CTC GGG AGC-3'

4. Vecteur d'expression d'ADN recombinant comprenant une séquence d'ADN recombinant suivant l'une quelconque des revendications 1 à 3.

5. Vecteur d'expression d'ADN recombinant comprenant une séquence d'ADN recombinant suivant l'une quelconque des revendications 1 à 3 et comprenant de plus une séquence promoteur et d'activation de traduction liée activement à la séquence d'ADN codant pour la DAC acétyltransférase.

6. Vecteur d'expression d'ADN recombinant suivant la revendication 5, dans lequel la séquence promoteur et d'activation de traduction est isolée de la même espèce que celle de laquelle la séquence d'ADN codant pour la DAC acétyltransférase est isolée.

7. Hôte eucaryotique ou procaryotique transformé avec un vecteur d'expression d'ADN recombinant suivant l'une quelconque des revendications 4 à 6.

8. Hôte procaryotique suivant la revendication 7, choisi parmi une espèce de champignons filamenteux, une espèce de streptomyces et une bactérie.

9. Hôte suivant la revendication 8, choisi parmi Acremonium Spp., Penicillium spp., E. coli et Bacillus spp.

10. Hôte suivant la revendication 9, choisi parmi Acremonium chrysogenum et Penicillium chrysogenum.

11. Procédé d'expression d'activité de DAC acétyltransférase chez un hôte eucaryotique ou procaryotique comprenant :
a) la transformation de la cellule hôte avec un vecteur d'expression d'ADN recombinant qui comprend (i) une séquence de contrôle d'expression qui fonctionne dans la cellule hôte et (ii) une séquence d'ADN codant pour la DAC acétyltransférase suivant l'une ou l'autre des revendications 1 et 2, qui est activement liée à la séquence de contrôle d'expression, et
b) la culture de la cellule hôte transformée dans l'étape (a) sous des conditions qui permettent l'expression d'activité de DAC acétyltransférase.

12. Procédé suivant la revendication 11, dans lequel la cellule hôte est choisie parmi Acremonium Spp., Penicillium spp., E. coli et Bacillus spp.

13. Procédé suivant la revendication 12, dans lequel la cellule hôte est choisie parmi Acremonium chrysogenum et Penicillium chrysogenum.

## Revendications (Revendications pour l'(les) Etat(s) contractant(s) suivant(s): ES, GR)

1. Procédé de préparation d'un vecteur d'expression d'ADN recombinant, comprenant la ligation ensemble d'une séquence d'ADN recombinant ou d'un fragment de restriction de celle-ci contenant un gène codant lors de l'expression pour une activité de DAC acétyltransférase obtenable d'Acremonium chrysogenum, ladite séquence d'ADN recombinant codant pour une protéine comprenant la structure amino terminale de : 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 mais ne contenant pas de gène codant lors de l'expression pour le polypeptide de DAOCS/DACS natif, et une séquence d'ADN qui code pour une séquence promoteur et d'activation de traduction activement liée à la séquence d'ADN codant pour la DAC acétyltransférase.

2. Procédé de préparation d'un vecteur d'expression d'ADN recombinant, comprenant la ligation ensemble d'une séquence d'ADN recombinant ou d'un fragment de restriction de celle-ci contenant le gène codant lors de l'expression pour une activité de DAC acétyltransférase obtenable d'Acremonium chrysogenum, ladite séquence d'ADN recombinant codant pour une protéine comprenant la structure amino terminale de : 
NH2-Met Leu Pro Ser Ala Gln Val Ala Arg Leu Lys Pro Asp Pro Phe Pro
Pro Ser Leu Ser Pro Ile Pro His Gly Ala Val Thr Phe Ala Ala Leu
Ala Pro Cys His Asn Leu Pro Ile Phe Ser Ser Arg Gln Met Leu Arg
Asp Ser Leu Thr Tyr Ser His Thr Ser Pro Thr Met Ser Pro Gln Ile
Ala Asn Arg Phe Glu Ala Ser Leu Asp Ala Gln Asp Ile Ala Arg Ile
Ser Leu Phe Thr Leu Glu Ser Gly Val Ile Leu Arg Asp Val Pro Val
Ala Tyr Lys Ser Trp Gly Arg Met Asn Val Ser Arg Asp Asn Cys Val
Ile Val Cys His Thr Leu Thr Ser Ser Ala His Val Thr Ser Trp Trp
Pro Thr Leu Phe Gly Gln Gly Arg Ala Phe Asp Thr Ser Arg Tyr Phe
Ile Ile Cys Leu Asn Tyr Leu Gly Ser...
 mais ne contenant pas le gène codant lors de l'expression pour une activité de DAOCS/DACS, et une séquence d'ADN qui code pour une séquence promoteur et d'activation de traduction liée activement à la séquence d'ADN codant pour la DAC acétyltransférase.

3. Procédé suivant l'une ou l'autre des revendications 1 et 2, dans lequel la séquence promoteur et d'activation de traduction est isolée de la même espèce que celle de laquelle la séquence d'ADN codant pour la DAC acétyltransférase est isolée.

4. Procédé suivant l'une quelconque des revendications 1 et 3, dans lequel la séquence d'ADN recombinant comprend la séquence nucléotidique de :

5'-ATG CTG CCG TCG GCC CAA GTG GCC CGT CTA AAG CCG GAC CCC TTT CCC
CCG AGT CTC TCC CCG ATC CCG CAC GGG GCC GTC ACT TTC GCT GCC CTC
GCT CCT TGT CAT AAC CTA CCT ATA TTC TCA TCC CGG CAA ATG CTG CGG
GAT AGC CTC ACC TAC AGC CAC ACG TCG CCC ACC ATG TCG CCT CAG ATC
GCC AAT CGC TTC GAG GCT TCG CTA GAT GCC CAA GAC ATA GCC AGA ATA
TCG CTC TTC ACA CTG GAA TCT GGC GTC ATC CTT CGC GAT GTA CCC GTG
GCA TAC AAA TCG TGG GGT CGC ATG AAT GTC TCA AGG GAT AAC TGC GTC
ATC GTC TGC CAC ACC TTG ACG AGC AGC GCC CAT GTC ACC TCG TGG TGG
CCC ACA CTG TTT GGC CAA GGC AGG GCT TTC GAT ACC TCT CGC TAC TTC
ATC ATC TGC CTA AAT TAT CTC GGG AGC-3'

5. Procédé d'expression d'une activité de DAC acétyltransférase chez un hôte eucaryotique ou procaryotique comprenant :
a) la transformation de la cellule hôte avec un vecteur d'expression d'ADN recombinant qui comprend (i) une séquence de contrôle d'expression qui fonctionne dans la cellule hôte et (ii) une séquence d'ADN codant pour la DAC acétyltransférase suivant l'une ou l'autre des revendications 1 et 2, qui est activement liée à la séquence de contrôle d'expression, et
b) la culture de la cellule hôte transformée dans l'étape (a) sous des conditions qui permettent l'expression d'activité de DAC acétyltransférase.

6. Procédé suivant la revendication 5, dans lequel la cellule hôte est choisie parmi Acremonium spp., Penicillium spp., E. coli et Bacillus spp.

7. Procédé suivant la revendication 6, dans lequel la cellule hôte est choisie parmi Acremonium chrysogenum et Penicillium chrysogenum.
